# FASCICULE DE BREVET EUROPEEN

(11) **EP 3 582 238 B1**
(45) Date de publication et mention de la délivrance du brevet: **30.12.2020**
(21) Numéro de dépôt: 19179145.8
(22) Date de dépôt: 07.06.2019
(51) Int. Cl.: H01F 7/02, G01R 33/383, G11C 11/02, A61N 2/06, H01F 3/10

(54) **STRUCTURE MAGNÉTIQUE CYLINDRIQUE DE TYPE HALBACH DIPOLAIRE**
ZYLINDRISCHE MAGNETISCHE STRUKTUR VOM TYP HALBACH-DIPOL
CYLINDRICAL MAGNETIC STRUCTURE SUCH AS A DIPOLAR HALBACH ARRAY

(30) Priorité: 11.06.2018 FR 1855073
(43) Date de publication de la demande: 18.12.2019
(73) Titulaire: Commissariat à l'Energie Atomique et aux Energies Alternatives, 75015 Paris (FR)
(72) Inventeur: DELAUNAY, Marc, 38054 GRENOBLE (FR)
(74) Mandataire: Cabinet Camus Lebkiri

(56) Documents cités:
- FR-A- 1 372 136
- US-A- 5 148 138
- SCHLUETER R D ET AL: "Magic Angle Rotating Field NMR/MRI Magnet for In Vivo Monitoring of Tissue", IEEE TRANSACTIONS ON APPLIED SUPERCONDUCTIVITY, IEEE SERVICE CENTER, LOS ALAMITOS, CA, US, vol. 17, no. 2, 1 juin 2008 (2008-06-01), pages 864-867, XP011214820, ISSN: 1051-8223
- JACHMANN R ET AL: "Multipole shimming of permanent magnets using harmonic corrector rings", REVIEW OF SCIENTIFIC INSTRUMENTS, AIP, MELVILLE, NY, US, vol. 78, no. 3, 30 mars 2007 (2007-03-30), pages 35115-35115, XP012103867, ISSN: 0034-6748, DOI: 10.1063/1.2713438

## Description

### DOMAINE TECHNIQUE

La présente invention concerne une structure magnétique cylindrique de type Halbach dipolaire permettant de produire une zone de champ magnétique sensiblement homogène, c'est-à-dire avec des lignes de champ magnétique sensiblement parallèles et sensiblement constantes en module. On entend par « sensiblement » des valeurs de module à +/- 5% et des variations d'orientation de lignes à +/- 1°.

L'invention trouve des applications dans tous les domaines nécessitant des structures magnétiques de grandes dimensions avec une zone de champ magnétique sensiblement homogène. Elle trouve en particulier des applications pour la production de mémoires magnétiques de type MRAM. Elle trouve également des applications dans le domaine médical pour la destruction de cellules cancéreuses par vibrations magnéto-mécaniques et pour l'imagerie par résonance magnétique nucléaire (RMN).

### ETAT DE LA TECHNIQUE

Il est connu d'utiliser des structures magnétiques cylindriques de type Halbach dipolaire pour générer des lignes de champ magnétique d'une grande homogénéité, c'est-à-dire des lignes de champ parallèles dont les modules sont sensiblement constants.

Il est, en particulier, connu d'utiliser des structures magnétiques cylindriques en aimants permanents de grandes dimensions pour différentes applications telles que la production de mémoires magnétiques de type MRAM (Magnetic Random Access Memory, en termes anglo-saxons) pour orienter parallèlement les domaines ferromagnétiques des électrodes (états parallèles/antiparallèles séparés par une barrière tunnel) par un recuit dans un champ magnétique homogène et de forte intensité. Le document « Magnetoresistive Random Access Memory» de D.Apalkov, B. Dieny et J.M. Slaughter, dans Proceeding of the IEEE, Vol.104, No10, Octobre 2016, décrit un exemple de réalisation d'une telle mémoire MRAM.

Pour une telle application, la structure magnétique cylindrique doit généralement être de grandes dimensions et présenter un rayon intérieur de plusieurs dizaines de centimètres. Un exemple d'une telle structure est décrit notamment dans l'article « Development of world'slargest Halbach - type magnetic circuit » de Shin-Etsu dans Rare Earth Magnets, Japan. La structure décrite dans cet article présente un diamètre intérieur de 50cm, un diamètre extérieur de 140cm, une largeur de 100cm et un poids de 10T. Une telle structure, particulièrement lourde et encombrante, permet la production d'un champ magnétique constant à 2.8% i.e. 1.05T+/-0.0294T ou de 1.02T à 1.08T.

Il est également connu d'utiliser des structures magnétiques cylindriques en aimants permanents de grandes dimensions pour des applications médicales telles que la cancérologie. Dans une telle application, la structure magnétique cylindrique comporte généralement un diamètre intérieur de 40 à 80cm pour permettre de traiter des patients (passage du corps ou au moins la tête pour le traitement du glioblastome) et produit un champ magnétique homogène de 30mT à 1 T suivant les cas. La structure magnétique cylindrique est mise en rotation à faible fréquence (typiquement 20HZ) afin de détruire les cellules cancéreuses par vibration magnéto-mécanique des microparticules magnétiques greffées sur des cellules, comme cela est décrit dans les documents suivants : « Triggering the apoptosis of targeted human renal cancer cells by the vibration of anisotropic magnetic particles attached to the cell membrane », S. Leulmi et al., Nanoscale, 2015,7,15904-15914 et « Rotating magnetic field induced oscillation of magnetic particles for in vivo mechanical destruction of malignant glioma », Y. Cheng et al., Journal of Controlled Release, 223 (2016) 75-84.

Des structures magnétiques cylindriques à champ homogène fixe ou en rotation peuvent aussi être utilisée dans l'imagerie par résonance magnétique nucléaire (RMN) comme décrit notamment dans les documents suivants : « System and Method for providing a rotating magnetic field », R. D. Schlueter and T. F. Budinger, United States Patent n° US2008/0024130 A1, Jan.31,2008 et « Permanent magnet ring assembly for NMR imaging system », S. Beer, U.S. Patent n° 4,703,276, Oct. 27,1987.

D'autres exemples de structures magnétiques cylindriques connues pour produire une zone de champ magnétique homogène sont décrites dans les documents « The ideal dimensions of a Halbach cylinder of finite length », R. Bjork, Journal of Applied Physics, Vol.109(1), 013915, 2011, «Optimization and improvement of halbach cylinder design » de R.Bjork et al., Journal of Applied Physics 104, 013910 (2008), « Dipolar Halbach Magnet Stacks Made from Identically Shaped Permanent Magnets for Magnetic Resonance », H. Soltner and P. Blümler, Concepts in Magnetic Resonance Part A, DOI 10.1002/cmr.a, «Magic Angle Rotating Field NMR/MRI Magnet for In Vivo Monitoring of Tissue», R. D. Schlueter and T. F. Budinger, IEEE Transactions on Applied Superconductivity, vol. 18, no. 2 (June 2008), «Multipole shimming of permanent magnets using harmonic corrector rings», R. C. Jachmann et al, Review of Scientific Instruments 78, 035115 (2007) et le brevet FR1372136.

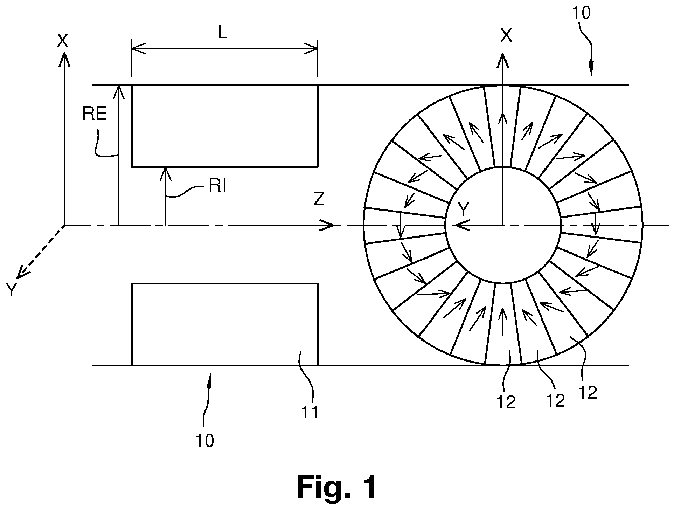

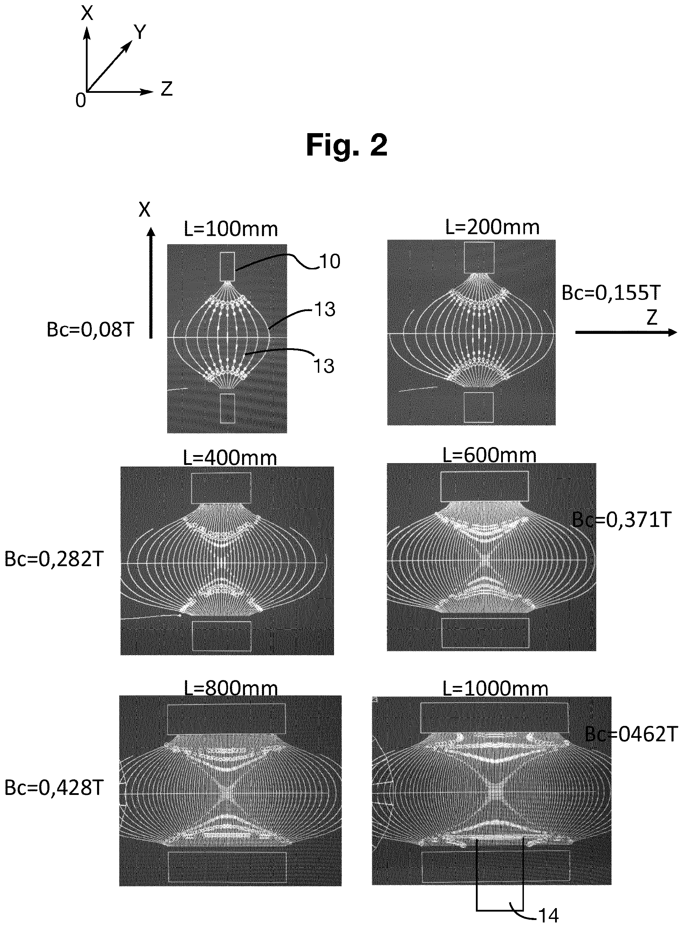

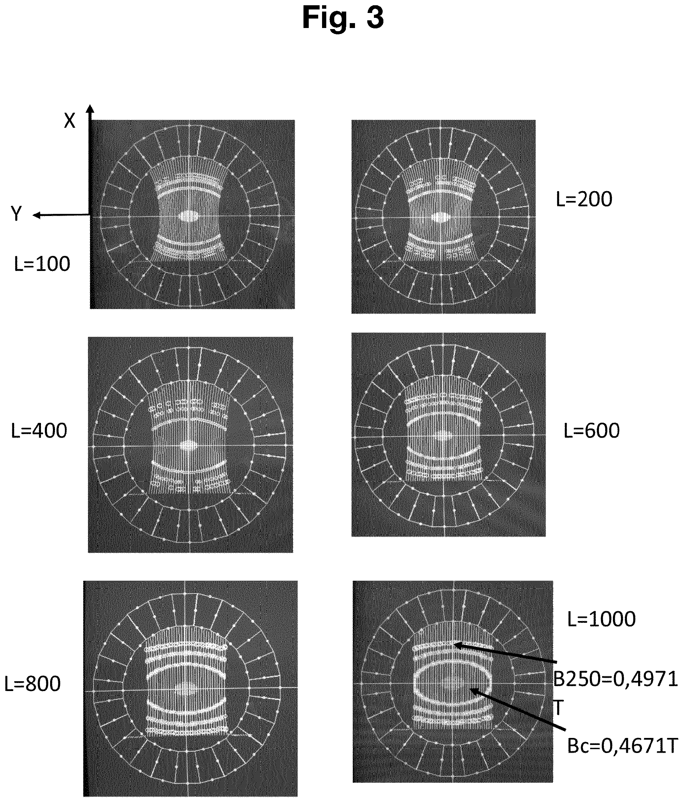

Un exemple d'une structure magnétique cylindrique classique est représentée selon une vue en coupe longitudinale et une vue en coupe diamétrale sur la figure 1. Cette structure magnétique cylindrique 10 comporte un cylindre 11 de rayon intérieur RI, de rayon extérieur RE et de largeur L. Ce cylindre 11 est constitué d'une pluralité d'aimants 12 montés en couronne, contiguement les uns aux autres, deux aimants contigus présentant des orientations de moments magnétiques différents. Une telle structure magnétique cylindrique 10 produit un champ magnétique comme montré sur les cartographies des figures 2 et 3. En particulier, la figure 2 représente des cartographies, dans un plan XZ d'un repère tridimensionnel OXYZ et la figure 3 représente des cartographies, dans le plan XY du même repère, de structures magnétiques cylindriques dont les largeurs L varient entre 100mm et 1000mm. Ces cartographies montrent que le champ magnétique se propageant suivant la direction X est de forme ovoïde avec des lignes de champ 13 courbes qui s'étendent, suivant la direction Z, en partie en extérieur du cylindre 10. La figure 2 montre qu'une zone de champ magnétique homogène ne peut être obtenue suivant la direction Z que pour une structure avec une largeur conséquente. En effet, pour une largeur L = 100mm, la zone homogène est quasi-inexistante. Une largeur L = 1000mm permet de produire la zone homogène 14 dans laquelle les lignes de champ 13 sont sensiblement parallèles et de module constant.

Toutefois, la quantité de lignes de champ intégrée dans la zone homogène 14 est petite en regard du nombre total de lignes de champs produites, un grand nombre de lignes de champs sortant du volume de la structure magnétique cylindrique. En effet, en traçant le rapport du champ magnétique au centre au champ hors axe à une distance considérée (par exemple 250mm), il apparait qu'une longueur d'environ 1 mètre est nécessaire pour obtenir une uniformité de champ à environ 5%.

Ainsi, quelle que soit l'application considérée, la structure magnétique cylindrique doit nécessairement être de grandes dimensions pour produire une zone de champ magnétique homogène suivant les trois directions X, Y et Z. Or, non seulement une structure magnétique cylindrique de grandes dimensions est lourde (plusieurs tonnes) et encombrante (volume supérieur au m3) mais elle nécessite aussi un très gros volume d'aimants dont l'assemblage est complexe et couteux. En outre, pour des applications où la structure magnétique cylindrique doit être rotative, les moteurs assurant leur rotation doivent être particulièrement performants (puissants) ; ils sont donc particulièrement onéreux.

### RESUME DE L'INVENTION

Pour répondre aux problèmes évoqués ci-dessus d'encombrement, de masse, de difficulté de mise en œuvre et de coût, le demandeur propose une structure magnétique cylindrique comportant trois cylindres de révolution de type Halbach dipolaire, alignés les uns avec les autres et permettant d'obtenir une zone de champ magnétique homogène optimisée.

L'invention concerne une structure magnétique cylindrique de révolution selon la revendication 1 produisant, dans un espace interne de ladite structure, un champ magnétique sensiblement homogène dans toutes les directions d'un repère orthonormé tridimensionnel (OXYZ) c'est-à-dire avec des lignes de champ magnétique sensiblement parallèles et sensiblement constantes en module . Cette structure se caractérise par le fait qu'elle comporte trois cylindres de révolution, creux, de type Halbach dipolaire, alignés les uns avec les autres suivant un même axe de révolution (Z), le premier, le deuxième et le troisième cylindres étant agencés comme suit :
- les premier, deuxième et troisième cylindres (110, 120, 130) sont disjoints,
- le deuxième cylindre est positionné entre le premier et le troisième cylindres,
- les premier, deuxième et troisième cylindres comportent des rayons extérieurs (REA, REB, REC) sensiblement identiques,
- la largeur (LB) du deuxième cylindre (120) est inférieure d'environ 10% aux largeurs (LA, LC) des premier et troisième cylindres, et
- le premier et le troisième cylindres comportent des rayons intérieurs (RIA, RIC) sensiblement identiques, inférieurs au rayon intérieur du deuxième cylindre (RIB),
- au moins un des trois cylindres (110, 120, 130) ayant un rayon intérieur (RI) supérieur ou égal à 250mm,
- le premier et le troisième cylindres comportent des largeurs (LA, LC) sensiblement identiques, et
- les premier, deuxième et troisième cylindres comportent chacun un plan médian, le plan médian du deuxième cylindre étant à une distance sensiblement égale du plan médian du premier cylindre et du plan médian du troisième cylindre.

Les rayons extérieurs des premier, deuxième et troisième cylindres ainsi que les rayons intérieurs et largeurs des premier et troisième cylindres sont identiques avantageusement à 5% près.

Cette structure magnétique cylindrique permet d'obtenir une zone de champ magnétique homogène à +/-5% et pouvant même atteindre un niveau d'homogénéité de +/-1% sur un grand volume, dans les trois directions X, Y et Z du repère orthonormé OXYZ et avec une masse de cylindre réduite.

On appelle « zone de champ magnétique homogène » un ensemble de lignes de champ magnétique sensiblement parallèles les unes aux autres et ayant un module de champ sensiblement identique.

Dans la suite de la description, la structure magnétique cylindrique à trois cylindres de l'invention sera appelée indifféremment structure magnétique cylindrique, structure magnétique ou simplement structure. De même, les lignes de champ magnétique et les modules de champ magnétique seront appelés indifféremment lignes de champ ou module de champ.

De façon avantageuse, chacun des premier, deuxième et troisième cylindres de la structure magnétique cylindrique comporte un nombre n d'aimants permanents, avec n > 2, et avantageusement supérieur ou égale à 6, disposés en couronne et orientés de sorte que les moments magnétiques de deux aimants contigus forment un angle de 360°/(n/2) dans un plan (OXY) du repère tridimensionnel (OXYZ).

De façon avantageuse, chacun des premier, deuxième et troisième cylindres de la structure comporte 24 aimants permanents disposés en couronne et orientés de sorte que les moments magnétiques de deux aimants contigus forment un angle de 30° dans le plan (OXY).

La structure magnétique cylindrique selon l'invention peut comporter une ou plusieurs des caractéristiques suivantes :
- les distances (dAB, dBC) entre respectivement les plans médians des premier et deuxième cylindres et les plans médians des deuxième et troisième cylindres répondent à l'équation : RIA/dAB =RIA/dBC=1.5 +/- 20%.
- le rayon intérieur (RIB) du deuxième cylindre (B) est supérieur aux rayons intérieurs des premier et troisième cylindres (RIA, RIC) avec : RIB = RIA + ΔR, où ΔR = 40mm +/-10%.
- les distances (dAB, dBC) entre les plans médians de chacun des premier, deuxième et troisième cylindres sont optimales lorsque RIA/dAB = RIA/dBC = 1,5 +/- 5%.
- les lignes de champ homogènes étant parallèles et à modules sensiblement constants, la valeur du module des lignes de champ varie en fonction de la dimension des rayons extérieurs des cylindres (REA, REB, REC).
- les lignes de champ homogènes étant parallèles et à modules sensiblement constants, la valeur du module des lignes de champ varie en fonction des largeurs des premier, deuxième et troisième cylindres (LA, LB, LC).

On appelle « plan médian » d'un cylindre de la structure selon l'invention le plan parallèle au plan (XZ) du repère OXYZ qui coupe diamétralement le cylindre et passe par le milieu de la largeur dudit cylindre.

Dans la suite de la description, des dimensions et distances seront définies comme étant « égale(s) » ou « sensiblement égale(s) », étant entendu que cette égalité peut être approximative, à quelques millimètres près.

### BREVE DESCRIPTION DES FIGURES

D'autres avantages et caractéristiques de l'invention apparaîtront à la lecture de la description, illustrée par les figures dans lesquelles :

La figure 1, déjà décrite, représente des vues en coupes longitudinale et diamétrale d'une structure magnétique cylindrique selon l'art antérieur.

Les figures 2 et 3, déjà décrites, représentent des cartographies dans des plans, respectivement XZ et XY, des champs magnétiques produits par des structures magnétiques cylindriques selon l'art antérieur.

La figure 4 représente un exemple d'un cylindre de la structure magnétique cylindrique selon l'invention.

Les figures 5A et 5B représentent des vues en coupes longitudinale et diamétrale de la structure magnétique cylindrique selon l'invention.

Les figures 6A, 6B, 6C et 6D représentent des exemples, respectivement, des lignes de champ magnétique et des isovaleurs du module du champ d'une structure selon l'invention dans les plans XY, XZ et les plans BX, BZ et leur représentation graphique.

La figure 6E représente deux exemples de lignes de champ magnétique et d'isovaleurs du module du champ de la structure magnétique cylindrique selon l'invention, dans les plans XY et XZ.

Les figures 7A et 7B représentent des exemples de profil de champs magnétiques pour, respectivement, une structure magnétique cylindrique selon l'art antérieur et une structure magnétique cylindrique selon l'invention, à masse totale équivalente.

Les figures 8A, 8B représentent des exemples de cartographies des isovaleurs du champ magnétique d'une structure magnétique cylindrique selon l'invention en fonction du rayon intérieur du deuxième cylindre.

La figure 9 représente des exemples de lignes de champ magnétique et d'isovaleurs du module du champ de la structure magnétique cylindrique selon l'invention, à zone de champ homogène optimisée pour différentes valeurs du rayon extérieur, du rayon intérieur et de la distance entre cylindres.

La figure 10 représente des exemples d'isovaleurs du champ magnétique du deuxième cylindre dans le plan XZ pour différentes valeurs de son rayon intérieur et pour différentes largeurs des cylindres.

La figure 11 représente graphiquement une variation optimisée de Xmax et du champ magnétique constant de la figure 10 en fonction de la largeur des cylindres et pour différentes valeurs du rayon intérieur du deuxième cylindre.

La figure 12 représente des exemples de lignes de champ magnétique et d'isovaleurs du module du champ de la structure magnétique cylindrique de l'invention, dans le plan XY, pour différentes valeurs de rayon extérieur.

### DESCRIPTION DETAILLEE D'AU MOINS UN MODE DE REALISATION

Un exemple de réalisation d'une structure magnétique cylindrique comportant trois cylindres alignés est décrit en détail ci-après, en référence aux dessins annexés. Cet exemple illustre les caractéristiques et avantages de l'invention. Il est toutefois rappelé que l'invention ne se limite pas à cet exemple.

Sur les figures, les éléments identiques sont repérés par des références identiques. Pour des questions de lisibilité des figures, les échelles de taille entre éléments représentés ne sont pas respectées.

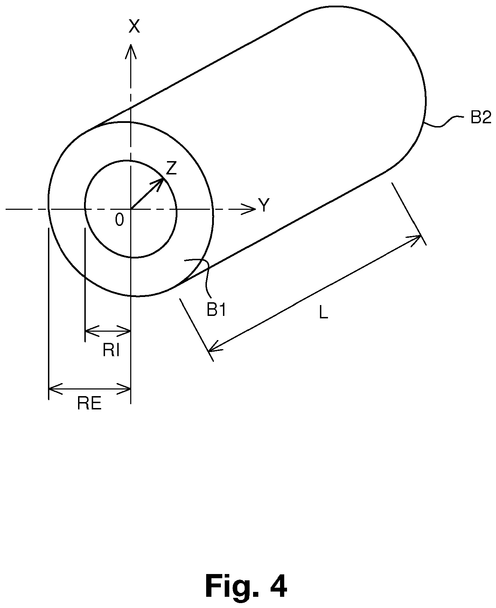

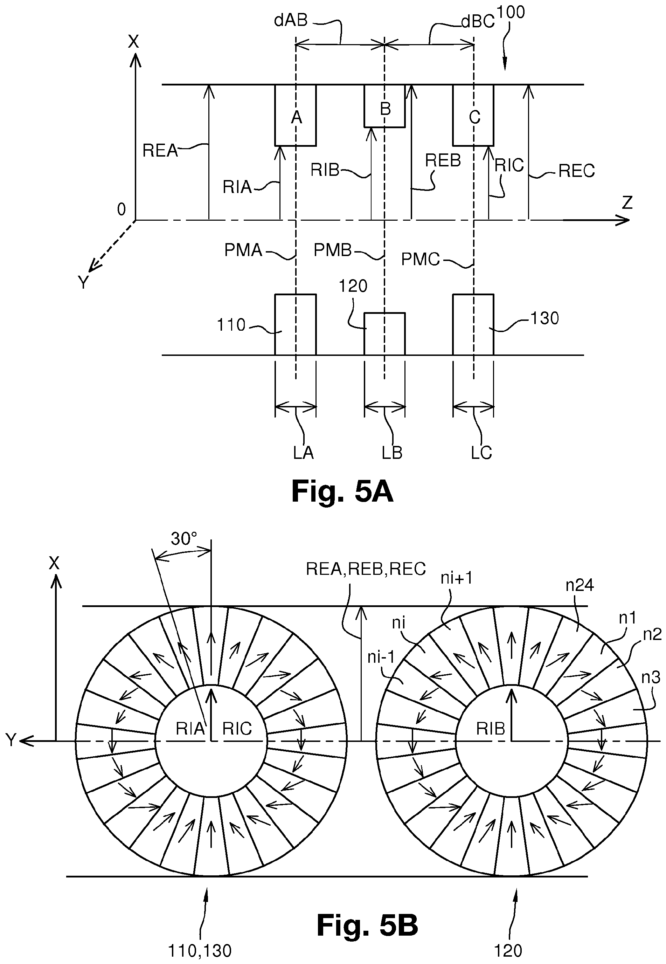

La figure 5A représente une vue en coupe longitudinale d'une structure magnétique cylindrique à trois cylindres selon l'invention. La figure 5B représente une vue en coupe diamétrale de chacun des trois cylindres de la structure magnétique cylindrique selon l'invention. Cette structure magnétique cylindrique 100 comporte un premier cylindre 110, appelé aussi cylindre A, un deuxième cylindre 120, appelé aussi cylindre B et un troisième cylindre 130, appelé aussi cylindre C. Chacun de ces cylindres est un cylindre creux, comme représenté sur la figure 4, monté autour d'un axe de révolution Z. Chaque cylindre est défini par un rayon intérieur RI, un rayon extérieur RE et une largeur L, la largeur L étant la distance séparant les deux bases circulaires parallèles B1, B2 du cylindre.

Comme représenté sur les figures 5A, 5B, les trois cylindres 110, 120, 130 sont montés ordonnés et alignés autour du même axe de révolution Z du repère tridimensionnel OXYZ. Autrement dit, les trois cylindres 110, 120, 130 sont montés de sorte que le deuxième cylindre 120 est positionné entre le premier et le troisième cylindres 110, 130, l'une des bases du deuxième cylindre 120 étant en regard d'une des faces du premier cylindre 110 et l'autre face du deuxième cylindre 120 étant en regard d'une des faces du troisième cylindre 130.

Chacun des trois cylindres 110, 120, 130 de la structure magnétique cylindrique est un cylindre magnétique de type Halbach dipolaire comprenant une pluralité d'aimants permanents nᵢ dont le moment magnétique - c'est-à-dire l'orientation d'aimantation - de deux aimants contigus, par exemple ni et nᵢ₋₁, forme un angle de 360°/(n/2). Le nombre d'aimants ni peut varier. Le module du champ magnétique au centre est maximum pour un nombre infini d'aimants et il diminue quand le nombre d'aimants diminue. Dans l'exemple représenté sur les figures 5A, 5B, et considéré par la suite, le nombre d'aimants est de 24, chaque aimant ayant une orientation magnétique décalée de 30° par rapport à l'aimant contigu. Ce nombre de 24 aimants offre un champ magnétique à 96-98% du champ optimum et un montage et une réalisation mécanique facilités.

Les trois cylindres 110, 120, 130 ont des rayons extérieurs RE identiques. Le rayon extérieur REA du premier cylindre 110, le rayon extérieur REB du deuxième cylindre 120 et le rayon REC du troisième cylindre 130 peuvent être, par exemple, compris entre 500mm et 700mm.

Les premier et troisième cylindres 110, 130 présentent un rayon intérieur RI identique. Autrement dit RIA = RIC, où RIA est le rayon intérieur du premier cylindre 110 et RIB est le rayon intérieur du troisième cylindre 130. Le deuxième cylindre 120 présente un rayon intérieur RIB supérieur aux rayons intérieurs RIA, RIC des premier et troisième cylindres 110, 130. Le rayon intérieur RIB du deuxième cylindre 120 peut être défini, par exemple, par : RIB (mm) = RIA(mm) + 40mm. Ainsi, les premier et troisième cylindres peuvent avoir un rayon intérieur RIA, RIC, par exemple, compris entre 250mm et 500mm et le deuxième cylindre un rayon intérieur RIB, par exemple, compris entre 290mm et 540mm. De façon optimale, l'un au moins des trois cylindres a un rayon intérieur RI supérieur ou égal à 250mm.

Les premier et troisième cylindres 110, 130 présentent une largeur L identique. Autrement dit LA = LC, où LA est la largeur du premier cylindre 110 et LC est la largeur du troisième cylindre 130. Selon certains modes de réalisation, le deuxième cylindre 120 présente une largeur LB inférieure à la largeur LA, LC des premier et troisième cylindres 110, 130. La largeur LB du deuxième cylindre 120 peut être définie, par exemple, par : LB(mm) = LA(mm) - 10%. Selon un exemple, la largeur LB du deuxième cylindre 120 peut être, par exemple, comprise entre 56mm et 170mm et la largeur LA, LC des premier et troisième cylindres 110, 130 comprises entre 60mm et 180mm.

Les trois cylindres 110, 120, 130 sont positionnés à distance sensiblement égale les uns des autres. Autrement dit, la distance dAB séparant le plan médian PMA du premier cylindre 110 et le plan médian PMB du deuxième cylindre 120 est égale, à un delta près, à la distance dBC séparant le plan médian PMB du deuxième cylindre 120 et le plan médian PMC du troisième cylindre 130, comme représenté sur la figure 5A. Plus précisément, les distances dAB et dBC entre les plans médians des trois cylindres 110, 120, 130 peuvent être définies par : RIA/dAB = RIA/dBC = 1,5 +/- 10%.

Comme représenté sur la figure 5A, les trois cylindres peuvent être distants, ou disjoints, les uns des autres. Autrement dit les cylindres A, B, C peuvent ne pas être accolés les uns aux autres. Ils peuvent, par exemple, être positionnés à distance les uns des autres de sorte que : $dAB > LA / 2 + LB / 2$ $dBC > LB / 2 + LC / 2$ $RIA / dAB = RIA / dBC = 1,5 + / - 20 % , et avantageusement + / - 5 % .$et avantageusement +/-5%.

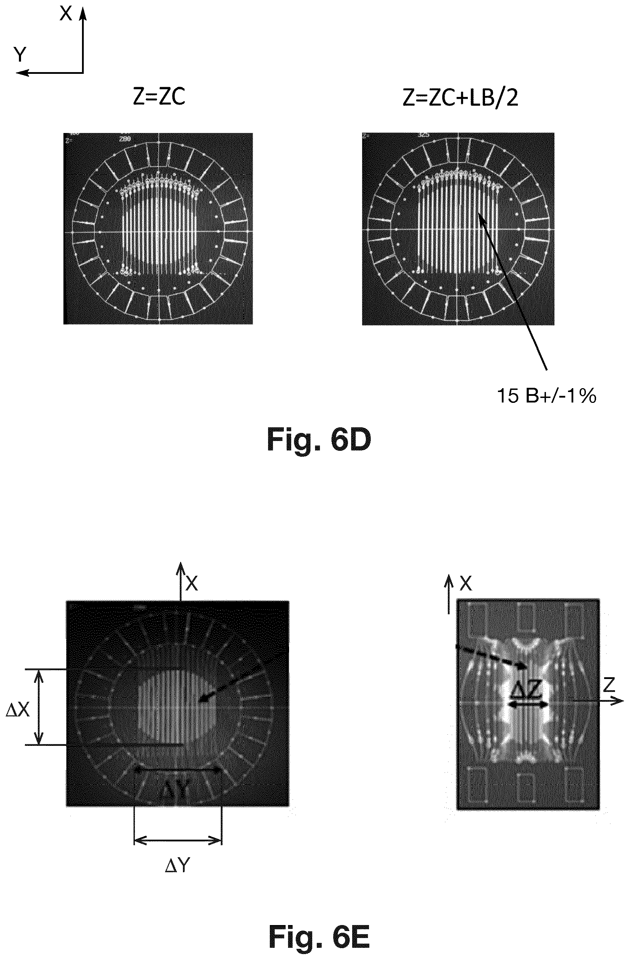

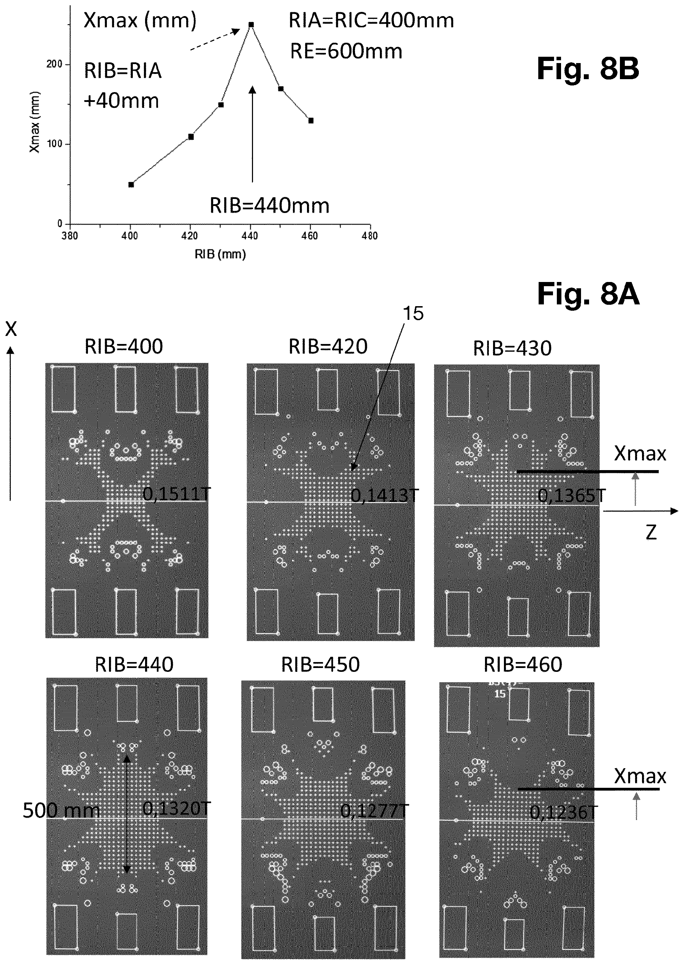

L'ajustement des rayons intérieurs RIA et RIB, avec RIA < RIB permet d'optimiser l'homogénéité du champ magnétique, cette homogénéité étant optimisée pour RIB (mm) = RIA (mm) + 40mm, comme montré dans les exemples des figures 8A et 8B. L'homogénéité du champ magnétique dans un volume ΔX.ΔY.ΔZ peut être quantifiée à +/-1% par rapport aux dimensions de la structure cylindrique en obtenant ΔX≥RIB, ΔY≥RIB et ΔZ≥RIB/2. Un exemple des intervalles ΔX, ΔY et ΔZ est représenté sur la figure 6E.

La structure magnétique cylindrique 100 telle que décrite précédemment permet de limiter la propagation des lignes de champ magnétique en extérieur de cylindre et produire une zone de champ homogène optimale, c'est-à-dire la plus large possible dans les 3 directions X, Y et Z, tout en réduisant la masse des aimants permanents de la structure. Cette zone de champ homogène est définie par des lignes de champ qui sont parallèles et dont les modules sont identiques - à quelques pourcents près (par exemple 1 à 2%) - dans les directions X, Y et Z du repère OXYZ. Ces modules de champ, lorsqu'ils sont identiques, sont appelés des isovaleurs.

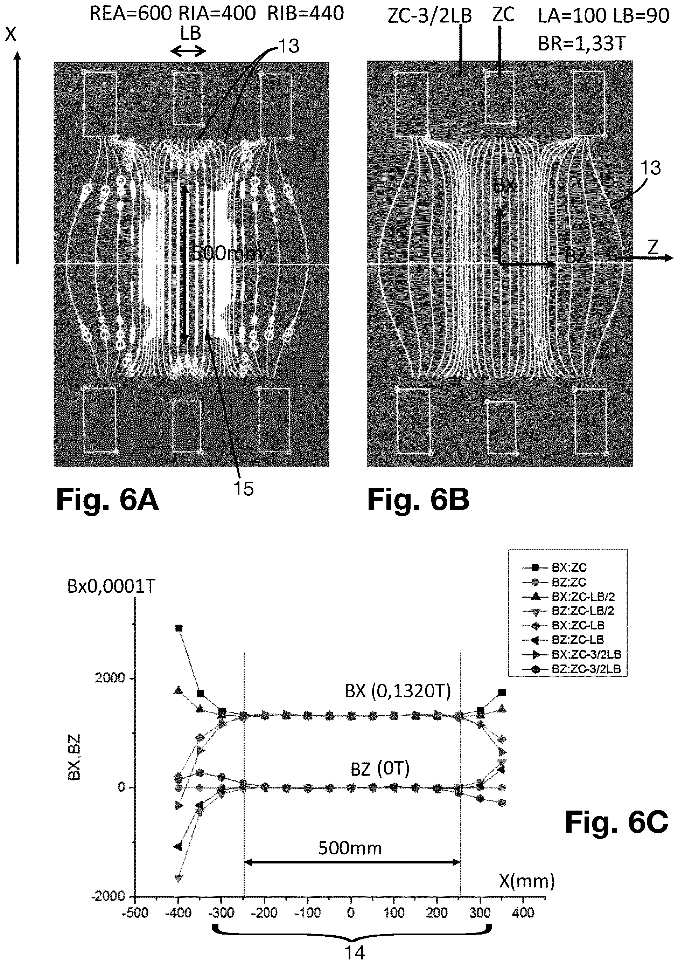

La figure 6A représente des exemples de lignes de champ 13 et d'isovaleurs 15 du champ magnétique obtenu avec la structure magnétique cylindrique à 24 aimants décrite ci-dessus, dans le plan XZ, lorsque :
- les trois cylindres ont un rayon extérieur RE égal à 600mm,
- les premier et troisième cylindres 110, 130 ont un rayon intérieur de 400mm et une largeur de 100mm,
- le deuxième cylindre 120 a un rayon intérieur de 440mm et une largeur de 90mm.

La figure 6D représente les lignes de champ magnétique et les isovaleurs, dans le plan XY, pour deux valeurs différentes de Z. La figure 6B représente les composantes BX et BZ du champ magnétique B suivant l'axe X pour différentes valeurs de Z. Sur un intervalle ΔX=500mm et ΔZ>250mm, la structure magnétique cylindrique ci-dessus permet d'obtenir le champ magnétique *̅B̅*̅=BXᵢ avec BX=0.1320T. La figure 6C représente, sous forme graphique, les lignes de champs 13 et les isovaleurs 15 dans la zone de champ homogène 14.

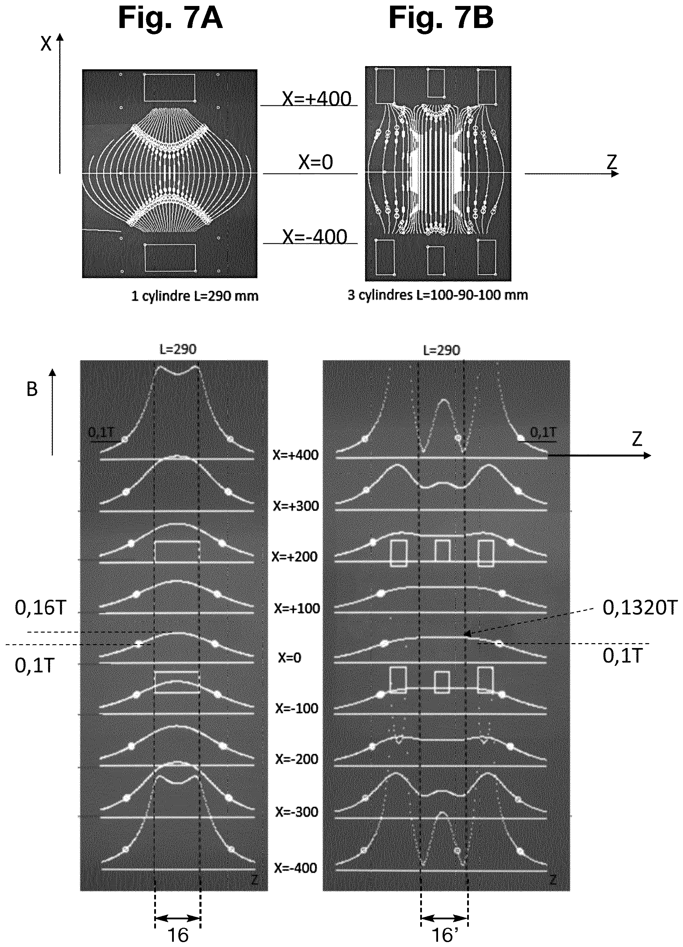

Les figures 7A et 7B représentent différents exemples de profils de champs magnétiques B suivant la direction Z dans un plan central où Y = 0 et pour différentes valeurs en mm de X. La figure 7A montre des exemples de champ B obtenus avec une structure à cylindrique unique de l'art antérieur, tandis que la figure 7B montre les champs obtenus, dans des conditions identiques, avec la structure magnétique cylindrique de l'invention. Une comparaison des deux figures 7A et 7B montre que le champ B produit par le cylindre unique présente une variation B(Z) du champ B dans la direction Z avec un maximum intrinsèque dans la zone centrale référencée 16, alors que la structure à trois cylindres génère un palier à champ B constant pour X variant entre -250mm et + 250mm. L'homme du métier comprendra que l'existence de ce palier dans les exemples de la figure 7B est à l'origine d'une augmentation de la zone de champ homogène par rapport aux exemples de la figure 7A.

La figure 8A représente des exemples d'isovaleurs 15 dans le plan XZ pour des rayons intérieurs RIB du deuxième cylindre 120 variant entre 400mm et 460mm. La détermination du rayon interne RIB optimal du deuxième cylindre 120 par rapport aux rayons internes RIA et RIC des premier et troisième cylindres 110, 130 correspond à l'obtention d'une zone de champ magnétique constant la plus grande possible suivant les 3 dimensions X, Y, Z - et en particulier dans le plan XZ - c'est-à-dire la valeur maximale de X (Xmax). En traçant la valeur de Xmax en fonction du rayon RIB, on obtient une valeur optimale : RIB = RIA + 40mm, comme représenté sur la figure 8B.

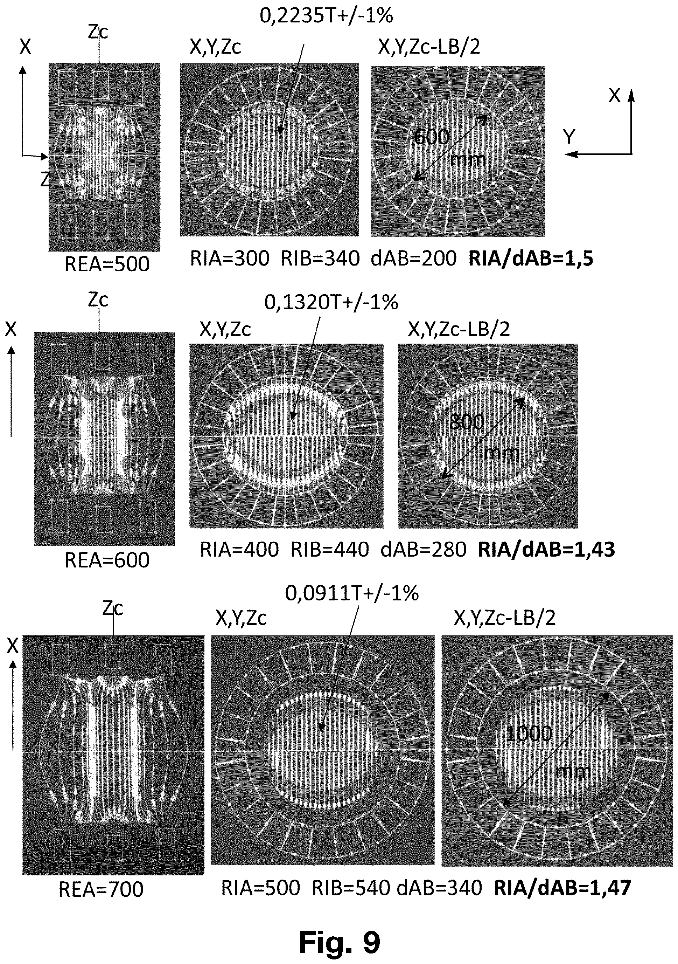

D'une façon plus générale, en faisant varier les rayons intérieurs RIA, RIC et RIB ainsi que la distance entre cylindre dAB, dBC, on obtient les paramètres qui permettent d'obtenir une zone homogène optimale : $RIB = RIA + 40 mm$ $RIA / dAB = 1,5 + / - 5 %$ La figure 9 représente des exemples de lignes de champ 13 et d'isovaleurs du module du champ pour une structure magnétique cylindrique à 3 cylindres à zone de champ homogène optimisées pour différentes valeurs de rayon extérieur RE, de rayon intérieur RI et de distance entre cylindre dAB, dBC, c'est-à-dire des valeurs vérifiant les deux équations ci-dessus.

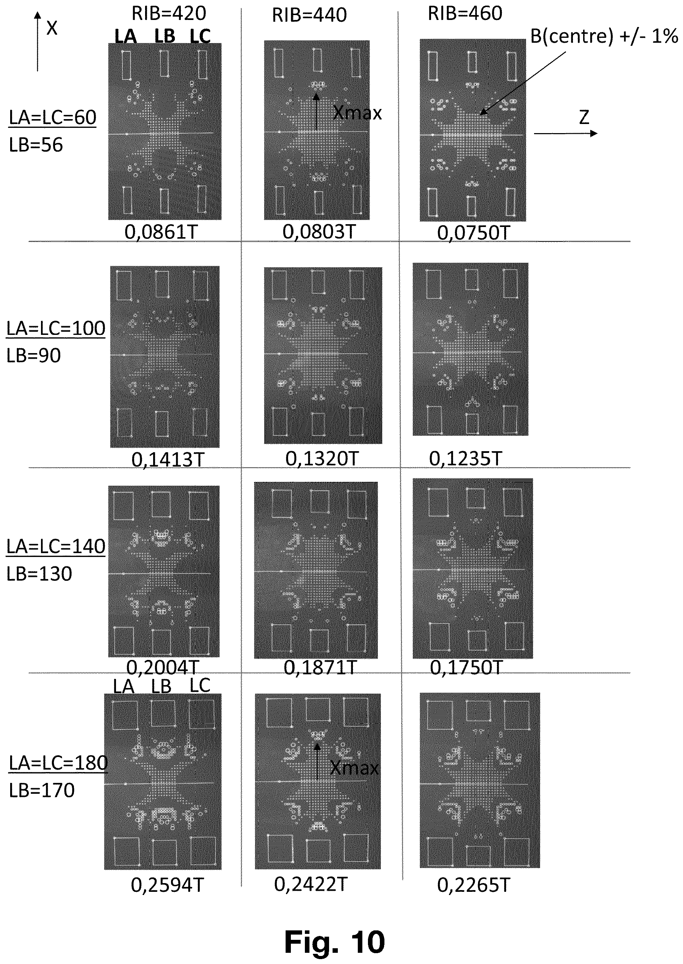

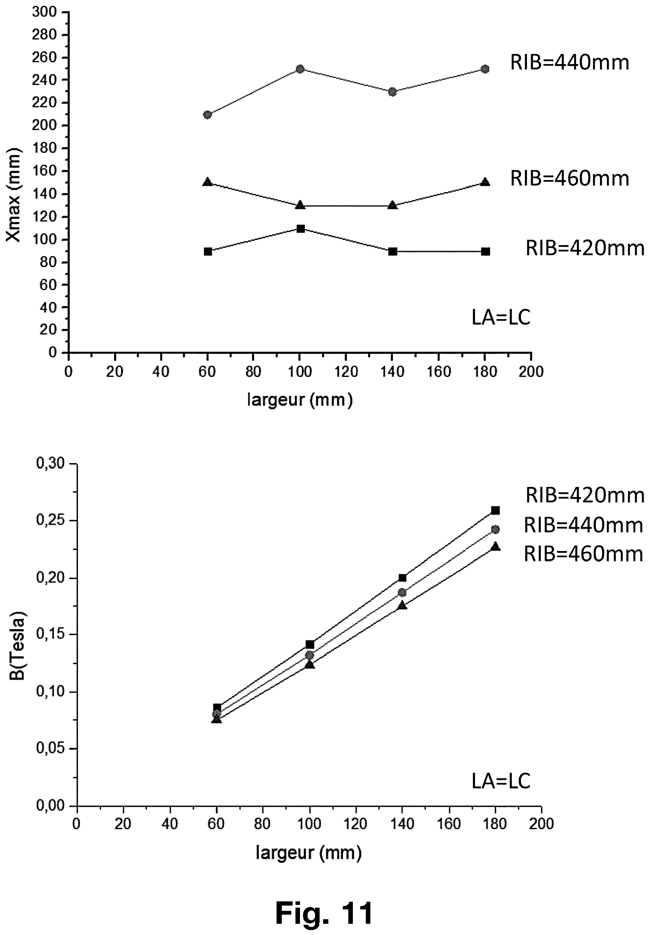

La figure 10 représente plusieurs exemples de cartographies d'isovaleurs du champ magnétique B dans le plan XZ pour différentes valeurs du rayon interne RIB du deuxième cylindre 120 qui vérifient RIB = RIA + 40mm et lorsque la largeur du deuxième cylindre varie entre 56mm et 170mm. Ces différents exemples montrent que la valeur du module du champ augmente, ou au contraire diminue, lorsque les largeurs LA, LB, LC des premier, deuxième et troisième cylindres augmente ou, respectivement, diminue. La figure 11 représente graphiquement les variations de Xmax optimisé et du champ magnétique B constant de la figure 10 en fonction de la largeur LA des premier et troisième cylindres pour différentes valeurs du rayon intérieur RIB du deuxième cylindre. Ces cartographies et graphiques montrent que la détermination des rayons intérieurs RIA, RIC, RIB n'est pas modifiée par la variation des largeurs LA, LB, LC des cylindres et que l'homogénéité et la constance du champ magnétique sont indépendantes des largeurs des cylindres. Autrement dit, l'augmentation (respectivement la diminution) de la largeur des cylindres 110, 120, 130 permet d'augmenter (respectivement de diminuer) le module du champ magnétique à l'intérieur de la structure magnétique cylindrique à trois cylindres sans modifier l'homogénéité et la constance du champ magnétique dans un volume ΔX.ΔY.ΔZ.

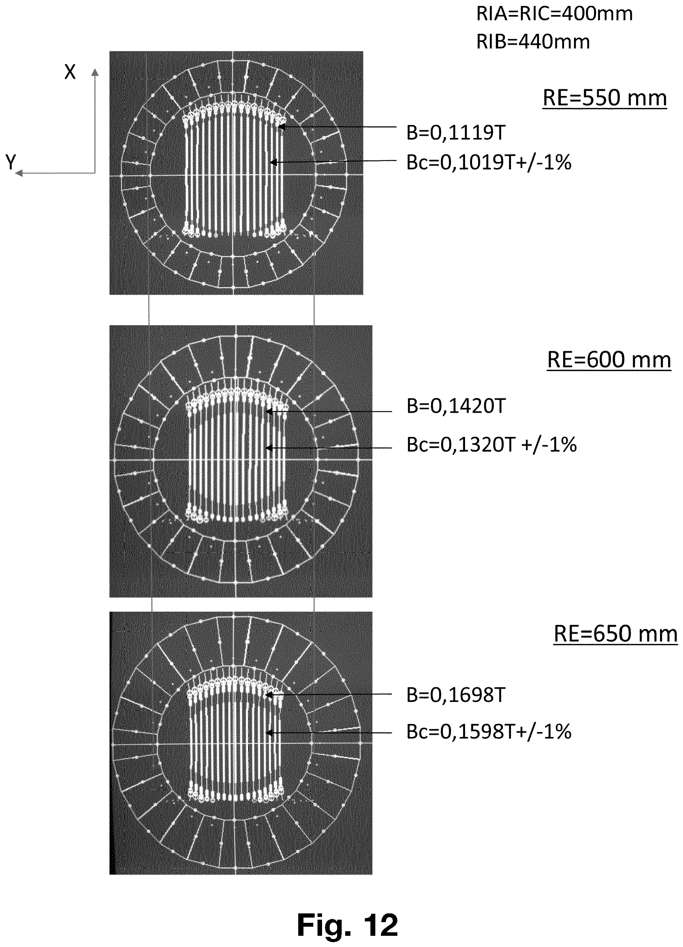

La figure 12 représente des exemples de lignes de champ magnétique et d'isovaleurs du module du champ de la structure magnétique cylindrique à trois cylindres de l'invention, dans le plan XY, en faisant varier le rayon extérieur RE de 550mm à 650 mm et avec des rayons intérieurs RIA=RIC=400mm et RIB=440mm. Ces différents exemples montrent que la valeur du champ augmente, ou au contraire diminue, lorsque les rayons extérieurs REA, REB, REC des premier, deuxième et troisième cylindres augmente ou, respectivement, diminue. Ces exemples montrent en outre que la détermination des rayons intérieurs RIA, RIC, RIB n'est pas modifiée par la variation du rayon extérieur RE des cylindres et que l'homogénéité et la constance du champ magnétique sont indépendantes du rayon extérieur. Autrement dit, l'augmentation (respectivement la diminution) du rayon extérieur des cylindres 110, 120, 130 permet d'augmenter (respectivement de diminuer) le module du champ magnétique à l'intérieur de la structure magnétique cylindrique à trois cylindres sans modifier l'homogénéité et la constance du champ magnétique dans un volume ΔX.ΔY.ΔZ.

Bien que décrit à travers un certain nombre d'exemples, variantes et modes de réalisation, la structure magnétique cylindrique selon l'invention comprend divers variantes, modifications et perfectionnements qui apparaîtront de façon évidente à l'homme du métier, étant entendu que ces variantes, modifications et perfectionnements font partie de la portée de l'invention.

## Revendications

1. Structure magnétique cylindrique de révolution (100) produisant, dans un espace interne de ladite structure, un champ magnétique (13) sensiblement homogène dans toutes les directions d'un repère orthonormé tridimensionnel (OXYZ),
**caractérisée en ce qu'**elle comporte trois cylindres de révolution (110, 120, 130), creux, de type Halbach dipolaire, alignés les uns avec les autres suivant un même axe de révolution (Z), le premier (110), le deuxième (120) et le troisième (130) cylindres étant agencés comme suit :
- les premier, deuxième et troisième cylindres (110, 120, 130) sont disjoints,
- le deuxième cylindre (120) est positionné entre le premier et le troisième cylindres (110, 130),
- les premier, deuxième et troisième cylindres (110, 120, 130) comportent des rayons extérieurs (REA, REB, REC) sensiblement identiques,
- le premier et le troisième cylindres (110, 130) comportent des rayons intérieurs (RIA, RIC) sensiblement identiques, inférieurs au rayon intérieur (RIB) du deuxième cylindre (120),
- au moins un des trois cylindres (110, 120, 130) a un rayon intérieur (RI) supérieur ou égal à 250mm,
- le premier et le troisième cylindres (110, 130) comportent des largeurs (LA, LC) sensiblement identiques,
- la largeur (LB) du deuxième cylindre (120) est inférieure d'environ 10% aux largeurs (LA, LC) des premier et troisième cylindres, et
- les premier, deuxième et troisième cylindres (110, 120, 130) comportent chacun un plan médian (PMA, PMB, PMC), le plan médian (PMB) du deuxième cylindre (120) étant à une distance sensiblement égale du plan médian (PMA) du premier cylindre et du plan médian (PMC) du troisième cylindre.

2. Structure magnétique selon la revendication 1, **caractérisée en ce que** chacun des premier, deuxième et troisième cylindres (110, 120, 130) comporte un nombre n d'aimants permanents, avec n > 2 et avantageusement supérieur ou égale à 6, disposés en couronne et orientés de sorte que les moments magnétiques de deux aimants contigus forment un angle de 360°/(n/2) dans un plan (XY) du repère tridimensionnel (OXYZ).

3. Structure magnétique selon la revendication 2, **caractérisée en ce que** chacun des premier, deuxième et troisième cylindres (110, 120, 130) comporte 24 aimants permanents disposés en couronne et orientés de sorte que les moments magnétiques de deux aimants contigus forment un angle de 30° dans le plan (XY).

4. Structure magnétique selon l'une quelconque des revendications 1 à 3, **caractérisée en ce que** les distances (dAB, dBC) entre respectivement les plans médians (PMA, PMB) des premier et deuxième cylindres (110, 120) et les plans médians (PMB, PMC) des deuxième et troisième cylindres répondent à l'équation : RIA/dAB =RIA/dBC=1.5 +/- 20%.

5. Structure magnétique selon l'une quelconque des revendications 1 à 4, **caractérisée en ce que** le rayon intérieur (RIB) du deuxième cylindre (120) est supérieur aux rayons intérieurs (RIA, RIC) des premier et troisième cylindres (110, 130) avec : RIB = RIA + ΔR, où ΔR = 40mm +/-10%.

6. Structure magnétique selon l'une quelconque des revendications 1 à 5, **caractérisée en ce que** les distances (dAB, dBC) entre les plans médians de chacun des premier, deuxième et troisième cylindres (110, 120, 130) sont optimales lorsque RIA/dAB = RIA/dBC = 1,5 +/- 5%.

7. Structure magnétique selon l'une quelconque des revendications 1 à 6, dans laquelle les lignes de champ homogènes (13) sont parallèles et à modules sensiblement constants, **caractérisée en ce que** la valeur du module du champ est déterminée par la dimension des rayons extérieurs (REA, REB, REC) des premier, deuxième et troisième cylindres, la valeur du module du champ étant augmentée lorsque la dimension des rayons extérieurs (REA, REB, REC) des premier, deuxième et troisième cylindres augmente et diminuée lorsque la dimension des rayons extérieurs (REA, REB, REC) des premier, deuxième et troisième cylindres diminue.

8. Structure magnétique selon l'une quelconque des revendications 1 à 7, dans laquelle les lignes de champ homogènes (13) sont parallèles et à modules sensiblement constants, **caractérisée en ce que** la valeur du module du champ est déterminée par la largeur (LA, LB, LC) des premier, deuxième et troisième cylindres, la valeur du module du champ étant augmentée lorsque la largeur (LA, LB, LC) des premier, deuxième et troisième cylindres augmente et diminuée lorsque la largeur (LA, LB, LC) des premier, deuxième et troisième cylindres diminue.

## Patentansprüche

1. Zylindrische magnetische Drehstruktur (100), die in einem inneren Raum der Struktur ein in allen Richtungen eines dreidimensionalen orthonormierten Bezugssystems (OXYZ) etwa homogenes Magnetfeld (13) produziert,
**dadurch gekennzeichnet, dass** sie drei hohle Drehzylinder (110, 120, 130) vom dipolaren Typ Halbach aufweist, die miteinander gemäß derselben Drehachse (Z) aufgereiht sind, wobei der erste (110), der zweite (120) und der dritte (130) Zylinder wie folgt eingerichtet sind:
- der erste, zweite und dritte Zylinder (110, 120, 130) sind getrennt,
- der zweite Zylinder (120) ist zwischen dem ersten und dem dritten Zylinder (110, 130) positioniert,
- der erste, zweite und dritte Zylinder (110, 120, 130) weisen etwa identische Außenradien (REA, REB, REC) auf,
- der erste und der dritte Zylinder (110, 130) weisen etwa identische Innenradien (RIA, RIC) auf, die kleiner als der Innenradius (RIB) des zweiten Zylinders (120) sind,
- mindestens einer der drei Zylinder (110, 120, 130) hat einen Innenradius (RI) größer oder gleich 250 mm,
- der erste und der dritte Zylinder (110, 130) weisen etwa identische Breiten (LA, LC) auf,
- die Breite (LB) des zweiten Zylinders (120) ist zirka 10 % kleiner als die Breiten (LA, LC) des ersten und dritten Zylinders, und
- der erste, zweite und dritte Zylinder (110, 120, 130) weisen jeweils eine Mittelebene (PMA, PMB, PMC) auf, wobei die Mittelebene (PMB) des zweiten Zylinders (120) in einem Abstand ist, der etwa gleich der Mittelebene (PMA) des ersten Zylinders und der Mittelebene (PMC) des dritten Zylinders ist.

2. Magnetische Struktur nach Anspruch 1, **dadurch gekennzeichnet, dass** jeder von dem ersten, zweiten und dritten Zylinder (110, 120, 130) eine Anzahl n Dauermagnete aufweist mit n > 2 und in vorteilhafter Weise größer oder gleich 6, die kranzförmig angeordnet sind und derart ausgerichtet, dass die magnetischen Momente von zwei aneinanderliegenden Magneten einen Winkel von 360°/(n/2) in einer Ebene (XY) des dreidimensionalen Bezugssystems (OXYZ) bilden.

3. Magnetische Struktur nach Anspruch 2, **dadurch gekennzeichnet, dass** jeder von dem ersten, zweiten und dritten Zylinder (110, 120, 130) 24 Dauermagnete aufweist, die kranzförmig angeordnet sind und derart ausgerichtet, dass die magnetischen Momente von zwei aneinanderliegenden Magneten einen Winkel von 30° in der Ebene (XY) bilden.

4. Magnetische Struktur nach einem der Ansprüche 1 bis 3, **dadurch gekennzeichnet, dass** die Abstände (dAB, dBC) zwischen jeweils den Mittelebenen (PMA, PMB) des ersten und zweiten Zylinders (110, 120) und die Mittelebenen (PMB, PMC) des zweiten und dritten Zylinders der Gleichung RIA/dAB = RIA/dBC = 1,5 +/- 20 % entsprechen.

5. Magnetische Struktur nach einem der Ansprüche 1 bis 4, **dadurch gekennzeichnet, dass** der Innenradius (RIB) des zweiten Zylinders (120) größer als die Innenradien (RIA, RIC) des ersten und dritten Zylinders (110, 130) ist mit RIB = RIA + ΔR, wobei ΔR = 40 mm +/- 10 %.

6. Magnetische Struktur nach einem der Ansprüche 1 bis 5, **dadurch gekennzeichnet, dass** die Abstände (dAB, dBC) zwischen den Mittelebenen von jedem von dem ersten, zweiten und dritten Zylinder (110, 120, 130) optimal sind, wenn RIA/dAB = RIA/dBC = 1,5 +/- 5 %.

7. Magnetische Struktur nach einem der Ansprüche 1 bis 6, wobei die homogenen Feldlinien (13) parallel sind und mit etwa konstanten Modulen, **dadurch gekennzeichnet, dass** der Wert des Feldmoduls von der Abmessung der Außenradien (REA, REB, REC) des ersten, zweiten und dritten Zylinders bestimmt wird, wobei der Wert des Feldmoduls zunimmt, wenn die Abmessung der Außenradien (REA, REB, REC) des ersten, zweiten und dritten Zylinders zunimmt und abnimmt, wenn die Abmessung der Außenradien (REA, REB, REC) des ersten, zweiten und dritten Zylinders abnimmt.

8. Magnetische Struktur nach einem der Ansprüche 1 bis 7, wobei die homogenen Feldlinien (13) parallel sind und mit etwa konstanten Modulen, **dadurch gekennzeichnet, dass** der Wert des Feldmoduls von der Breite (LA, LB, LC) des ersten, zweiten und dritten Zylinders bestimmt wird, wobei der Wert des Feldmoduls zunimmt, wenn die Breite (LA, LB, LC) des ersten, zweiten und dritten Zylinders zunimmt und abnimmt, wenn die Breite (LA, LB, LC) des ersten, zweiten und dritten Zylinders abnimmt.

## Claims

1. Cylindrical magnetic structure of revolution (100) producing, in an internal space of said structure, a substantially homogeneous magnetic field (13) in all directions of a three-dimensional orthonormal coordinate system (OXYZ), **characterised in that** it comprises three cylinders of revolution (110, 120, 130), hollow, of the Halbach dipolar array type, aligned with one another according to a same axis of revolution (Z), with the first (110), the second (120) and the third (130) cylinders being arranged as follows:
- the first, second and third cylinders (110, 120, 130) are disjointed,
- the second cylinder (120) is positioned between the first and the third cylinders (110,130),
- the first, second and third cylinders (110, 120, 130) comprise external radiuses (REA, REB, REC) that are substantially identical,
- the first and the third cylinders (110, 130) comprise internal radiuses (RIA, RIC) that are substantially identical, less than the inner radius (RIB) of the second cylinder (120),
- at least one of the three cylinders (110, 120, 130) has an inner radius (RI) greater than or equal to 250mm,
- the first and the third cylinders (110, 130) comprise widths (LA, LC) that are substantially identical,
- the width (LB) of the second cylinder (120) is less than about 10% of the widths (LA, LC) for the first and third cylinders, and
- the first, second and third cylinders (110, 120, 130) each comprise a median plane (PMA, PMB, PMC), the median plane (PMB) of the second cylinder (120) being at a substantially equal distance from the median plane (PMA) of the first cylinder and from the median plane (PMC) of the third cylinder.

2. Magnetic structure according to claim 1, **characterised in that** each one of the first, second and third cylinders (110, 120, 130) comprises a number n of permanent magnets, with n > 2 and advantageously greater than or equal to 6, arranged in a ring and oriented in such a way that the magnetic moments of two contiguous magnets form an angle of 360°/(n/2) in a plane (XY) of the three-dimensional coordinate system (OXYZ).

3. Magnetic structure according to claim 2, **characterised in that** each one of the first, second and third cylinders (110, 120, 130) comprises 24 permanent magnets arranged in a ring and oriented in such a way that the magnetic moments of two contiguous magnets form an angle of 30° in the plane (XY).

4. Magnetic structure according to any of claims 1 to 3, **characterised in that** the distances (dAB, dBC) between respectively the median planes (PMA, PMB) of the first and second cylinders (110, 120) and the median planes (PMB, PMC) of the second and third cylinders satisfy the equation: RIA/dAB =RIA/dBC=1.5 +/- 20%.

5. Magnetic structure according to any of claims 1 to 4, **characterised in that** the inner radius (RIB) of the second cylinder (120) is greater than the internal radiuses (RIA, RIC) of the first and third cylinders (110, 130) with: RIB = RIA + ΔR, where ΔR = 40mm +/-10%.

6. Magnetic structure according to any of claims 1 to 5, **characterised in that** the distances (dAB, dBC) between the median planes of each one of the first, second and third cylinders (110, 120, 130) are optimal when RIA/dAB = RIA/dBC = 1.5 +/- 5%.

7. Magnetic structure according to any of claims 1 to 6, wherein the homogeneous field lines (13) are parallel and with substantially constant moduli, **characterised in that** the value of the modulus of the field is determined by the dimension of the external radiuses (REA, REB, REC) of the first, second and third cylinders, the value of the modulus of the field being increased when the dimension of the external radiuses (REA, REB, REC) of the first, second and third cylinders increases and decreased when the dimension of the external radiuses (REA, REB, REC) of the first, second and third cylinders decreases.

8. Magnetic structure according to any of claims 1 to 7, wherein the homogeneous field lines (13) are parallel and have substantially constant moduli, **characterised in that** the value of the modulus of the field is determined by the width (LA, LB, LC) of the first, second and third cylinders, the value of the modulus of the field being increased when the width (LA, LB, LC) of the first, second and third cylinders increases and decreased when the width (LA, LB, LC) of the first, second and third cylinders decreases.
